(19) Europäisches Patentamt / European Patent Office / Office européen des brevets

(11) **EP 3 224 836 B1**

(12) **EUROPEAN PATENT SPECIFICATION**

(45) Date of publication and mention
of the grant of the patent:
**25.12.2019 Bulletin 2019/52**

(51) Int Cl.:
*H01B 3/44* *(2006.01)*     *C08L 27/18* *(2006.01)*
*C08L 23/06* *(2006.01)*

(21) Application number: **14906839.7**

(22) Date of filing: **28.11.2014**

(86) International application number:
**PCT/CN2014/092558**

(87) International publication number:
**WO 2016/082212 (02.06.2016 Gazette 2016/22)**

(54) **PROCESS FOR FOAMING POLYOLEFIN COMPOSITIONS USING FLUORORESIN/CITRATE MIXTURE AS NUCLEATING AGENT**

VERFAHREN ZUM SCHÄUMEN VON POLYOLEFINZUSAMMENSETZUNGEN MIT FLUORHARZ-/CITRATMISCHUNG ALS NUKLEIERUNGSMITTEL

PROCÉDÉ DE MOUSSAGE DE COMPOSITIONS POLYOLÉFINIQUES FAISANT INTERVENIR UN MÉLANGE DE RÉSINE FLUORÉE ET DE CITRATE COMME AGENT DE NUCLÉATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.10.2017 Bulletin 2017/40**

(73) Proprietor: **Dow Global Technologies LLC Midland, MI 48674 (US)**

(72) Inventors:
• **SUN, Gangwei**
  **Shanghai 201210 (CN)**
• **ESSEGHIR, Mohamed**
  **Lawrenceville, New Jersey 08648-4844 (US)**
• **XU, Xian Min**
  **Shanghai 200136 (CN)**
• **KMIEC, Chester J.**
  **Phillipsburg, New Jersey 08865 (US)**

(74) Representative: **Boult Wade Tennant LLP Salisbury Square House 8 Salisbury Square London EC4Y 8AP (GB)**

(56) References cited:
**EP-A1- 1 935 931     WO-A1-2004/094526
US-A- 4 650 815     US-A- 5 225 107
US-A- 5 688 449     US-A1- 2014 030 521
US-B1- 6 492 596**

**Description**

FIELD OF THE INVENTION

This invention relates to a process of foaming compositions. In one aspect the invention relates to foaming polyolefin compositions using a fluororesin as a nucleating agent while in another aspect, the invention relates to the foamed composition made from the process. In yet another aspect, the invention relates to using the foamed compositions as an insulation layer in electric communication cables, particularly high frequency coaxial cables.

BACKGROUND OF THE INVENTION

Typically, the insulation layer of a high frequency telecom cable is produced by mixing a nucleating agent with a mixture of high density polyethylene (HDPE) and low density polyethylene (LDPE). The foamable materials are then extruded in the presence of a physical foaming agent, like gases such as nitrogen, carbon dioxide, chlorinated fluorocarbons, freons, helium, neon, argon, krypton, xenon, and radon, which is injected into the polymer melt inside of the extruder. Nucleating agents for the foaming can include but not limited to azodicarbonamide (ADCA) and 4,4'-oxybisbenzenesulfonylhydrazide (OBSH), which thermally decompose in an extruder and form a number of fine nuclei in the polymer melt. However, the byproducts of the decomposed ADCA and OBSH have a high polarity which are well known to have a significant negative effect on the electrical performance (dissipation factor) of the cable.

Compared to ADCA and OBSH, fluororesin powder, such as polytetrafluoroethylene (PTFE), is a nucleating agent that exhibits a significantly lesser effect on electrical performance and is free of the decomposition issues associated with ADCA and OBSH. PTFE has been and is currently used as a nucleating agent for foaming compositions for use as insulation in telecom cable but improvements are still desired, particularly with respect to dispersion of the nucleating agent within the foamable composition, i.e., the polymer matrix, and in the formation of small, uniformly sized cells within the foamed product.

The dispersion efficiency of nucleator in a polymer matrix is largely determined by the particle size and particle size distribution of the nucleator. USP 3,554,932A teaches that finely divided, solid fluororesins, such as PTFE, fluorinated ethylene-propylene (FEP), or particle carriers coated with a fluorocarbon functioned as nucleators for gas-injected, foamed thermoplastic. It also teaches that the particle size should not exceed 20 microns in diameter, and it should be used in an amount from 0.01% to 2% by weight.

US 2009/0018225 teaches a foaming composition comprising a polymer having a melting temperature above 250°C and an organic salt as the chemical foaming agent, the salt having a decomposition temperature above the melting point of the polymer. The organic salt is selected from the group consisting of citrate derivatives and tartrate derivatives or a mixture of the same.

CA2523861 A1 teaches a low loss foam composition and cable, such as a coaxial cable. The foam composition is formed by heating an olefinic polymer, such as a high density polyethylene, medium density polyethylene, low density polyethylene, linear low density polyethylene, polypropylene, or a combination thereof, into a molten state composition, optionally with a nucleating agent. The molten mixture is extruded under pressure through a die with a blowing agent comprising an atmospheric gas, such as carbon dioxide, nitrogen or air, and a co-blowing agent. The nucleating agent is selected from the group consisting of: azobisformamide, azodicarbonamide and sodium carbonate, with or without citric acid, talc, calcium carbonate, mica and combinations thereof.

SUMMARY OF THE INVENTION

In one embodiment the invention is a process of foaming a polyolefin composition using as a nucleator a combination of (A) a fluororesin, and (B) a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate.

In one embodiment the invention is a polyolefin foam made by a process for foaming a polyolefin composition using as a nucleator a combination of (A) a fluororesin, and (B) a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate.

In one embodiment the invention is a cable comprising an insulation layer comprising a foam made by a process of foaming a polyolefin composition using as a nucleator a combination of (A) a fluororesin, and (B) a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate.

## DETAILED DESCRIPTION OF THE PREFERRED EMBODIMENT

*Definitions*

Unless stated to the contrary, implicit from the context, or customary in the art, all parts and percents are based on weight and all test methods are current as of the filing date of this disclosure. For purposes of United States patent practice, the contents of any referenced patent, patent application or publication are incorporated by reference in their entirety (or its equivalent US version is so incorporated by reference) especially with respect to the disclosure of definitions (to the extent not inconsistent with any definitions specifically provided in this disclosure) and general knowledge in the art.

The numerical ranges in this disclosure are approximate unless otherwise indicated. Numerical ranges include all values from and including the lower and the upper values, in increments of one unit, provided that there is a separation of at least two units between any lower value and any higher value. As an example, if a compositional, physical or other property, such as, for example, tensile strength, elongation at break, etc., is from 100 to 1,000, then the intent is that all individual values, such as 100, 101, 102, etc., and sub ranges, such as 100 to 144, 155 to 170, 197 to 200, etc., are expressly enumerated. For ranges containing values which are less than one or containing fractional numbers greater than one (e.g., 1.1, 1.5, etc.), one unit is considered to be 0.0001, 0.001, 0.01 or 0.1, as appropriate. For ranges containing single digit numbers less than ten (e.g., 1 to 5), one unit is typically considered to be 0.1. These are only examples of what is specifically intended, and all possible combinations of numerical values between the lowest value and the highest value enumerated, are to be considered to be expressly stated in this disclosure. Numerical ranges are provided within this disclosure for, among other things, particle size and the amount of individual ingredients in a mixture.

"Comprising," "including," "having" and like terms are not intended to exclude the presence of any additional component, step or procedure, whether or not the same is specifically disclosed. In order to avoid any doubt, all processes claimed through use of the term "comprising" may include one or more additional steps, pieces of equipment or component parts, and/or materials unless stated to the contrary. In contrast, the term, "consisting essentially of' excludes from the scope of any succeeding recitation any other component, step or procedure, excepting those that are not essential to operability. The term "consisting of" excludes any component, step or procedure not specifically delineated or listed. The term "or," unless stated otherwise, refers to the listed members individually as well as in any combination.

"Composition"and like terms mean a mixture of two or more materials.

"Polyolefin composition" and like terms mean, in the context of this invention, a composition comprising at least one polyolefin.

"Interpolymer" means a polymer prepared by the polymerization of at least two different monomers. This generic term includes copolymers, usually employed to refer to polymers prepared from two different monomers, and polymers prepared from more than two different monomers, e.g., terpolymers, tetrapolymers, etc.

"Nucleator", "nucleating agent" and like terms mean, in the context of this invention, a substance, typically a small particle, that provides a nucleation site or location for bubble formation within a polymer melt. Nucleating agents are used to enhance the cell structure of foamed polymers.

"Agglomerate" and like terms mean a collection of two or more particles group together to constitute a whole. Agglomerates can be of various sizes. An agglomerate will always be larger than the particles from which it is made, but some particles not associated with a particular agglomerate can be larger than the agglomerate. In the practice of this invention, agglomerates are typically and preferably less than one micron is size, more preferably less than 0.5 micron and even more preferably less than 0.3 micron, in size.

"Particle" and like terms mean a unitary mass. Particles can be of various sizes. A fluororesin particle, e.g., a PTFE particle, is a unitary mass of fluororesin. Two or more fluororesin particles grouped together, i.e., in contact with one another, form a fluororesin agglomerate. The fluororesin particles of this invention are typically and preferably less than one micron is size, more preferably less than 0.5 micron and even more preferably less than 0.3 micron, in size.

"Unagglomerated particle" and like terms mean a particle not associated with another particle of like kind. Unagglomerated particles include both particles that have dissociated from an agglomerate, and particles that have not been associated with an agglomerate.

"Masterbatch" and like terms mean a concentrated mixture of additives in a carrier resin. In the context of this invention, a masterbatch comprises a concentrated mixture of fluororesin nucleator in a polyolefin resin. The masterbatch allows for an efficient addition and dispersion of the nucleator to and in the polyolefin. The manufacture and use of masterbatches are well known to those skilled in the art of manufacturing and fabricating plastics and foam articles.

*Polyolefins*

"Polyolefin" and like terms means a polymer derived from one or more simple olefin monomers, e.g., ethylene, propylene, 1-butene, 1-hexene, 1-octene and the like. The olefin monomers can be substituted or unsubstituted and if substituted, the substituents can vary widely. If the polyolefin is to contain unsaturation, then preferably at least one of the comonomers is at least one nonconjugated diene such as 1,7-octadiene, 1,9-decadiene, 1,11-dodecadiene, 1,13-tetradecadiene, 7-methyl-1,6-octadiene, 9-methyl-1,8-decadiene and the like. Many polyolefins are thermoplastic. Polyolefins include but are not limited to polyethylene, polypropylene, polybutene, polyisoprene and their various interpolymers.

According to the invention the polyolefin is at least one, preferably a blend of, high density polyethylene (HDPE) and low density polyethylene (LDPE). The HDPE resins that can be used in the practice of this invention are well known, commercially available, and can be prepared with either Ziegler-Natta, chromium-based, constrained geometry or metallocene catalysts in slurry reactors, gas phase reactors or solution reactors. HDPE, as used herein, is an ethylene-based homopolymer or interpolymer having a density of at least 0.94 g/cc, or from at least 0.94 g/cc to 0.98 g/cc, and a melt index from 0.1 g/10 min to 25 g/10 min.

HDPE can comprise ethylene and one or more $C_3$-$C_{20}$ $\alpha$-olefin comonomers. The comonomer(s) can be linear or branched. Nonlimiting examples of suitable comonomers include propylene, 1-butene, 1 pentene, 4-methyl-1-pentene, 1-hexene, and 1-octene. HDPE interpolymer includes at least 50 percent by weight units derived from ethylene, i.e., polymerized ethylene, or at least 70 percent by weight, or at least 80 percent by weight, or at least 85 percent by weight, or at least 90 weight percent, or at least 95 percent by weight ethylene in polymerized form.

In an embodiment, HDPE is a homopolymer or an ethylene/$\alpha$-olefin copolymer with a density from 0.94 g/cc to 0.98 g/cc, and a melt index from 0.1 g/10 min to 10 g/10 min. In an embodiment, the HDPE has a density from 0.960 g/cc to 0.980 g/cc, and a melt index from 0.1 g/10 min to 10 g/10 min. In an embodiment, HDPE has a density from 0.96 g/cc to 0.97 g/cc and a melt index from 0.1 g/10 min to 10 g/min. In an embodiment, the HDPE has a density from 0.96 g/cc to 0.98 g/cc and a melt index from 1.0 g/10 min to 10.0 g/10 min.

Nonlimiting examples of suitable, commercially available HDPE include but are not limited to DOW High Density Polyethylene resins and CONTINUUM™ and UNIVAL™ high density polyethylene resins, ELITE™ 5960G, HDPE KT 10000 UE, HDPE KS 10100 UE and HDPE 35057E, each available from The Dow Chemical Company Midland, Michigan, USA; SURPASS™ available from Nova Chemicals Corporation, Calgary, Alberta, Canada; BS2581 available from Borealis; Hostalen ACP 5831D available from Lyondell/Basell; RIGIDEX® HD5502S available from INEOS Olefins & Polymers Europe; SABIC®B5823 and SABIC®B5421 available from Sabic; and HDPE 5802 and BM593 available from Total.

The LDPE resins that can be used in the practice of this invention are also well known, commercially available, and made by any one of a wide variety of processes including, but not limited to, solution, gas or slurry phase, and high pressure tube or autoclave. The polyethylene also can be homogeneous or heterogeneous with respect to comonomer distribution. The homogeneous polyethylenes usually have an essentially uniform comonomer distribution. The heterogeneous polyethylenes, on the other hand, do not have a uniform comonomer distribution. In one embodiment the LDPE is a linear low density polyethylene (LLDPE). In one embodiment the LDPE is a very low density polyethylene (VLDPE).

The polyethylene can have a broad molecular weight distribution, characterized by a polydispersity (Mw/Mn) greater than 3.5, or a narrow molecular weight distribution, characterized by a polydispersity (Mw/Mn) in the range of about 1.5 to about 3.5. Mw is defined as weight average molecular weight, and Mn is defined as number average molecular weight. They can be a single type of polyethylene or a blend or mixture of more than one type of polyethylene. Thus, it may be characterized by either single or multiple DSC melting points. The polyethylenes can have a density in the range of 0.865 to 0.930 gram per cubic centimeter (g/cc), and preferably have a density in the range of 0.900 to 0.925 g/cc. They also can have a melt index (MI, $I_2$) in the range of 0.1 to 50 grams per 10 minutes (g/10 min). Typical catalyst systems, which can be used to prepare these polyethylenes, are magnesium/titanium based catalyst systems, which can be exemplified by the catalyst system described in USP 4,302,565 (heterogeneous polyethylenes); vanadium based catalyst systems such as those described in USP 4,508,842 (heterogeneous polyethylenes) and 5,332,793; 5,342,907; and 5,410,003 (homogeneous polyethylenes); a chromium based catalyst system such as that described in USP 4,101,445; a metallocene catalyst system such as that described in USP 4,937,299 and 5,317,036 (homogeneous polyethylenes); or other transition metal catalyst systems. Many of these catalyst systems are often referred to as Ziegler-Natta catalyst systems or Phillips catalyst systems. Catalyst systems, which use chromium or molybdenum oxides on silica-alumina supports, can be included here. Typical processes for preparing the polyethylenes are also described in the aforementioned patents. Typical *in situ* polyethylene blends and processes and catalyst systems for providing same are described in USP 5,371,145 and 5,405,901. The various polyethylenes can include low density homopolymers of ethylene made by high pressure processes (HP-LDPE), and high density polyethylene (HDPE) having a density greater than 0.940 g/cc. A conventional high pressure process is described in Introduction to Polymer Chemistry, Stille, Wiley and Sons, New York, 1962, pages 149 to 151. The high pressure processes are typically free radical initiated polymerizations conducted in a tubular reactor or a stirred autoclave. In the stirred autoclave, the pressure is in the range of about 10,000 to 30,000 psi (about 69 to about 207 MPa) and the temperature is in the range of about 175°C to about 250°C., and in the tubular reactor, the pressure is in the range of about 25,000 to about 45,000 psi (about 170 to about 310 MPa) and the temperature is in the range of about 200°C to about 350°C.

Commercially available LDPE resins include but are not limited to DOW Low Density Polyethylene resins available from The Dow Chemical Company such as DFDB-1258 NTand, in general, any fractional melt flow index (MFI) resin for use in heavy duty bags or agricultural films such as those available from Borealis, Basel, Sabic and others.

The HDPE/LDPE mixtures or blends of the present invention may be prepared by any suitable means known in the art such as, for example, dry blending in a pelletized form in desired proportions followed by melt blending in an apparatus such as a screw extruder or a BANBURY™ mixer. Dry blended pellets may be directly melt processed into a final solid state article by, for example, extrusion or injection molding. The blends may also be made by direct polymerization. Direct polymerization may use, for example, one or more catalysts in a single reactor or two or more reactors in series or parallel and vary at least one of operating conditions, monomer mixtures and catalyst choice.

The amount of HDPE in the polyolefin composition, based on the weight of the composition, is typically at least 45 weight percent (wt %), more typically at least 55 wt% and even more typically at least 60 wt%. The amount of HDPE in the polyolefin composition, based on the weight of the composition, typically does not exceed 95 wt%, more typically it does not exceed 85 wt% and even more typically it does not exceed 80 wt%.

The amount of LDPE in the polyolefin composition, based on the weight of the composition, is typically at least 4 weight percent (wt %), more typically at least 14 wt% and even more typically at least 19 wt%. The amount of LDPE in the polyolefin composition, based on the weight of the composition, typically does not exceed 54 wt%, more typically it does not exceed 44 wt% and even more typically it does not exceed 39 wt%.

The HDPE component of the blend can comprise two or more grades of HDPE, and the LDPE component of the blend can comprise two or more grades of LDPE. The HDPE/LDPE blend typically has an $I_2$ of 0.1 to 4 g/10 min, more typically 0.15 to 4 g/10 min.

*Nucleator*

*Fluororesin Component*

Fluororesin particles, particularly those of less than a micron in size, tend to agglomerate. Some commercially available fluororesin powders comprise a high concentration of agglomerates of at least 5 microns ($\mu$m) in size, e.g., diameter. Typically the size of the agglomerates range from 4 to 50 microns, more typically from 5 to 20 microns and even more typically from 5 to 15 microns. Typically, the amount of fluororesin agglomerates of at least 5 $\mu$m in size in these powders is at least 80%, more typically at least 82%, and even more typically at least 85%. These powders do not disperse well in many polyolefins, e.g., HDPE and/or LDPE.

While agglomerated fluororesin particles, i.e., agglomerates, as described above can be used in the practice of this invention,in one embodiment unagglomerated particles are used. In one embodiment the fluororesin components of the nucleators used in this invention are unagglomerated particles of less than a micron in size, or less than 0.5 micron in size,or less than 0.3 micron in size, which may be commingled with agglomerates that were either originally submicron in size or were reduced in size from greater than a micron to less than a micron.In one embodiment the fluororesin component of the nucleator used in the practice of the invention comprises less than 10 wt%, or 9wt%, or 8wt%, or 7wt%, or 6wt%, or 5wt%, or 4wt%, or 3 wt%, or 2wt%, or 1wt% of agglomerates greater than a micron in size, but the smaller the amount of such agglomerates, and thus the greater the amount of submicron particles and submicron agglomerates, the better the dispersion of the fluororesin in the polyolefin, and the more evenly distributed are the cell sizes in the foamed product.

Agglomerated particles can be separated from one another by any conventional means, e.g., grinding, mixing or stirring (typically at a relatively high speed), etc. In one embodiment a fluororesin comprising agglomerates of one micron or greater, typically of 3, or 4, or 5 microns or greater, is subjected to any procedure, treatment, etc. that will reduce the majority, preferably 60%, 70%, 80%, 90% or more, of the such agglomerates to either unagglomerated particles of less than a micron in size, or agglomerates of less than a micron in size before the nucleator is mixed with the polyolefin.

In one embodiment the fluororesin component of the nucleator used in the practice of this invention and comprising agglomerates of one micron or greater, typically of 3, or 4, or 5 microns or greater, is first mixed with the polyolefin, with or without the citrate mixture component of the nucleator, to form a masterbatch, and then the masterbatch is subjected to any procedure, treatment, etc. that will reduce the majority, preferably 60%, 70%, 80%, 90% or more, of the such agglomerates to either unagglomerated particles of less than a micron in size, or agglomerates of less than a micron in size. Typically the masterbatch comprises from 1 to 50, more typically from 5 to 50 and even more typically from 15 to 30 weight percent (wt%) fluororesin, and from 50 to 99, more typically from 60 to 95 and even more typically from 70 to 85 wt% polyolefin. After the masterbatch is subjected to the fluororesin size reduction procedure, treatment, etc., the masterbatch is mixed with the citrate mixture component of the nucleator (if it does not already comprise that component) and the polyolefin to be foamed under conditions and for a sufficient period of time to uniformly disperse the unagglomerated particles and agglomerates within the polyolefin before the start of the foaming process.

In one embodiment the fluororesin comprising agglomerates of one micron or greater, typically of 3, or 4, or 5 microns or greater, is first mixed with the polyolefin, with or without the citrate mixture component of the nucleator, in the amount desired for the practice of the foaming process, and then the polyolefin is subjected to any procedure, treatment, etc. for a sufficient amount of time that will both (1) reduce the majority, preferably 60%, 70%, 80%, 90% or more, of the such agglomerates to either unagglomerated particles of less than a micron in size, or agglomerates of less than a micron in size, and (2) substantially uniformly disperse these unagglomerated particles and reduced agglomerates within the polyolefin before the foaming process commences. The citrate component of the nucleator can be added to the polyolefin before, simultaneously with, or after the addition of the fluororesin, and before or after the agglomerates of the fluororesin are subjected to size reduction.

The nucleator, preferably PTFE comprising particles and agglomerates of less than a micron in size, can be added to the polyolefin composition comprising or consisting essentially of HDPE and LDPE, by any conventional means. The nucleator can be added neat, in combination with one or more other additives, e.g., antioxidant, cell stabilizer, etc., or as part of a masterbatch. The nucleator is mixed with the polyolefin composition to achieve an essentially homogeneous dispersion of nucleator in the polyolefin composition and to this end, batch mixing, e.g., through the use of a BUSS™ kneader, is typically preferred to mixing in an extruder. If the nucleator is first mixed with the polyolefin composition in an extruder, then it is typically added to the polyolefin composition prior to injection of the gas for foaming.

Particle size can be determined by any method known in the art. In one embodiment, the determination of particle size and proportion (% by number) of fluororesin powder can be determined as follows. A dispersion comprising a fluororesin powder obtained by a dispersing treatment for about 2 minutes under ultrasonication of about 35-40 kHz and ethanol, wherein the fluororesin powder is contained in an amount to make a laser permeation (proportion of output light to incident light) of the dispersion 70-95%, is subjected to a microtrack particle size analyzer under relative refraction (determination is done based on the ratio of diffraction ratio (about 0.99) of fluororesin powder to that of ethanol or according to the measure of the above-mentioned particle size analyzer which is the nearest to the ratio (e.g., 1.02)) and flow type cell measurement mode to determine particle size ($D_1$, $D_2$, $D_3$ ...) of individual particles and the number ($N_1$, $N_2$, $N_3$ ...) of particles having each particle size based on the optical diffraction of the laser. In this case, the particle size (D) of individual particles is automatically measured by the microtrack particle size analyzer wherein particles having various shapes are measured in terms of the diameters of the corresponding spheres. Therefore, the proportion (% by number) of the particle size $D_1$ is expressed by the percentage of the number of these particles ($N_1$) to the number of the entire particles ($\Sigma N$). The proportion of the particles having a particle size of 0.1-0.5 $\mu$m is expressed by the percentage of the number of the particles having a particle size of 0.1-0.5 .$\mu$m to the total number of the existing particles ($\Sigma N$). Similarly, the proportion of the particles having a particle size of not less than 5 $\mu$m is expressed by the percentage of the number of the particles having a particle size of not less than 5 $\mu$m to the total number of the existing particles ($\Sigma N$). On the other hand, the average particle size of the nucleator of the present invention can be calculated using the total number of existing particles ($\Sigma N$) and the total of the product of the cube of the particle size of respective particles and the total number of existing particles ($\Sigma ND^3$), according to the following formula

$$\text{Average Particle Size } (\mu m) = (\Sigma ND^3/\Sigma N)^{1/3}$$

Calculation of particle size is further illustrated in USP 6,121,335. The calculation of agglomerate size is determined in the same manner as that described above for particle size determination.

While the shape of the fluororesin particles and agglomerates is not particularly limited, it is preferable that the particles and agglomerates are primarily sphere-like in shape to produce a foam comprising fine cells and superior in uniform foaming.

*Citrate Mixture Component*

The citrate mixture comprises or consists essentially of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate. Of course, if the first component of the mixture is an alkali metal citrate, then the second component of the mixture is not an alkali metal citrate. Preferably the alkali metal in each of the compounds identified in the first and second components is sodium. Preferred citrate mixtures consist of citric acid and/or sodium citrate as the first component with sodium bicarbonate as the second component. The weight ratio of first component to second component of the citrate mixture is typically from 1:99 to 99:1, more typically from 20:80 to 80:20. Typically, the shape and size of the component parts of the citrate mixture are irregular and 2 to 50 microns, respectively.

*Fluororesin/Citrate Mixture*

The weight ratio of citrate mixture to fluororesin, preferably PTFE, is typically from 20:80 to 85:15 and even more typically from 50:50 to 80:20. Expressed as the amount of fluororesin and citrate mixture added to the polyolefin composition, sufficient fluororesin is added to the polyolefin composition to result in the fluororesin comprising from 0.01 to 1 wt%, typically from 0.05 to 0.6 wt% and more typically from 0.1 to 0.3 wt% of the polyolefin composition. Sufficient citrate mixture is added to the polyolefin composition to result in the fluororesin comprising from 300 parts per million (ppm) to 5,000 ppm, typically from 500 ppm to 5,000 ppm and more typically from 500 ppm to 3,000 ppm of the polyolefin composition.

The amount of the nucleator of this embodiment, i.e., fluororesin and citrate mixture, that is added to the polyolefin composition is typically from 0.01 to 1 wt%, more typically from 0.05 to 0.6 wt% and even more typically from 0.1 to 0.3 wt% based on the weight of the polyolefin composition. The manner of mixing the nucleator of this embodiment with the polyolefin composition is also as described above.

The use of the fluororesin/citrate mixture nucleator of this embodiment produces a higher performance product as compared to a product produced using a fluororesin, particularly PTFE, alone as the nucleator. The products exhibit enhanced properties in terms of expansion ratio, cell size and cell size uniformity as well as surface smoothness. In this hybrid nucleating agent, the fluororesin is the "passive" nucleating agent and citrate mixture is the "active" nucleating agent. The synergic effect between these two nucleating agents results in a higher nuclei density and smaller cell size as compared to processes using and products produced by the use of neat PTFE or neat citrate mixture, or its component parts, alone as the nucleating agent.

*Additives*

The polyolefin composition used in this invention may contain one or more additives as necessary or desired. Representative additives include but are not limited to, processing aids, lubricants, stabilizers (antioxidants), foaming aids, nucleating agents, surfactants, flow aids, viscosity control agents, coloring agents, copper inhibitors and the like. These additives can be added to the polymer(s) either before or during processing. The amount of any particular additive in the polyolefin composition is typically from 0.01 to 1 wt%, more typically from 0.01 to 0.5 wt% and even more typically from 0.01 to 0.3 wt%, and the total amount of additives in the polyolefin composition, if present at all, is typically from 0.01 to 5 wt%, more typically from 0.01 to 2 wt% and even more typically from 0.01 to 1 wt%.

*Foaming Agent*

The foaming agent is one or more suitable for the extrusion temperature, foaming conditions, foam forming method and the like. When an insulating foam layer in the final form is to be formed simultaneously with extrusion forming, for example, an inert gas such as nitrogen, a carbon gas (e.g., CO, $CO_2$, etc.), helium, argon and the like, hydrocarbon such as methane, propane, butane, pentane and the like, halogenated hydrocarbons such as dichlorodifluoromethane, dichloromonofluoromethane, monochlorodifluoromethane, trichloromonofluoromethane, monochloropentafluoroethane, trichlorotrifluoroethane and the like are used. The amount of the foaming agent to be used can vary. Typically, it is 0.001-0.1 part by weight, more typically 0.005-0.05 part by weight, per 100 parts by weight of the polyolefin composition to be foamed. The foaming agent may be mixed with an organic polymer to be foamed in advance or may be supplied into an extruder from a foaming agent supply opening formed on the barrel of the extruder.

*Foaming Process*

The polyolefin composition of this invention is foamed using known methods and known equipment. Typically, a foam is produced by extruding the polyolefin composition containing a nucleator using an extruder operated under foaming extrusion conditions, e.g., injection of a foaming agent while the composition is in a high pressure zone and then extruding the composition to a low pressure zone. Foaming process are further described by C.P. Park in Polyolefin Foam, Chapter 9, Handbook of Polymer Foams and Technology, edited by D. Klempner and K. C. Frisch, Hanser Publishers (1991).

The polyolefin composition of this invention is foamed using known methods and known equipment. Typically, a foam is produced by extruding the polyolefin composition containing a nucleator using an extruder operated under foaming extrusion conditions, e.g., injection of a foaming agent while the composition is in a high pressure zone and then extruding the composition to a low pressure zone. Foaming process are further described by C.P. Park in Polyolefin Foam, Chapter 9, Handbook of Polymer Foams and Technology, edited by D. Klempner and K. C. Frisch, Hanser Publishers (1991).

In one embodiment, a typical extrusion foaming process uses an atmospheric gas (e.g., $CO_2$) to produce a foamed cable insulation as described in CA 2 523 861 C, Low Loss Foam Composition and Cable Having Low Loss Foam Layer. Dissolution of the foaming gas into the polymer melt is governed by Henry's law as reported for example in the work of H. Zhang (below) and others. Solubility is a function of the saturation pressure and the Henry's law constant, which itself is a function of temperature. /Zhang_Hongtao_201011_MASc_thesis.pdf. Also see *Foam Extrusion: Principles and Practice* by Shau-Tarng Lee, editor. The MuCell® microcellular foam injection molding technology is an example of a commercially practiced foaming process, and it is described generally in USP 6,284,810.

Given the above on the importance of adequate pressure control during foaming extrusion, a suitable process would be the one commercially referred to as the MuCell process, in which adequate pressures are built *via* specific hardware design, for effective nucleation as reported in US 6,84,810B1. The method disclosed in this publication relies solely on high pressure drops (dP/dt) for self-nucleation of the foaming gas in the absence of an "auxiliary nucleating agent" (Col. 4, line 25-30).

*Embodiments of the Invention*

In one embodiment the polyolefin composition comprises at least two polyolefins.

In one embodiment the polyolefin composition consists of two polyolefins.

In one embodiment the polyolefins of the polyolefin composition are an HDPE and a LDPE.

In one embodiment the polyolefin composition includes at least one nucleator.

In one embodiment the polyolefin composition includes at least one of an antioxidant and a cell stabilizer.

In one embodiment the polyolefin composition comprises HDPE, LDPE, PTFE and a citrate mixture.

In one embodiment the polyolefin composition comprises HDPE, LDPE and a nucleator consisting of (A) PTFE, and (B) a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate.

In one embodiment the citrate mixture of the polyolefin composition of any of the preceding embodiments comprises sodium bicarbonate, sodium citrate and disodium hydrogen citrate.

In one embodiment the citrate mixture of the polyolefin composition of any of the preceding embodiments comprises sodium dihydrogen citrate and citric acid.

SPECIFIC EMBODIMENTS

The following experiments are provided to illustrate various embodiments of the invention. They are not intended to limit the invention as otherwise described and claimed. All numerical values are approximate.

*Examples 1-6 and Comparative Examples 1-4*

*Materials*

LDPE-1 is a low density polyethylene (LDPE) with an MI of 2.3 g/10 min (ASTM D-1238, (190°C./2.16 kg)) and a density of 0.92 g/cc (ASTM D-792).

PTFE is ZONYL™ MP 1400, a white, free-flowing PTFE with an average particle size of 10 μm and available from DuPont.

SONGNOX™ 1024 FG is 2',3-bis[[3-[3,5-di-tert-butyl-4-hydroxyphenyl]-propionyl]]propionohydrazide, an antioxidant available from Songwon International - Americas, Inc.

LDPE-2 is DFDB-1258 NT, a low density polyethylene (LDPE) with an MI of 6 g/10 min (ASTM D-1238, (190°C./2.16 kg)) and a density of 0.922 g/cc (ASTM D-792) available from The Dow Chemical Company.

HDPE-1 is DGDA-6944 NT, a high density polyethylene (HDPE) with an MI of 8 g/10 min (ASTM D-1238, (190°C./2.16 kg)) and a density of 0.965 g/cc (ASTM D-792) available from The Dow Chemical Company.

CM-1 is HYDROCEROL™ CF, a mixture of sodium bicarbonate (majority component), sodium citrate and disodium hydrogen citrate available from Clariant Corporation.

CM-2 is a mixture sodium dihydrogen citrate and citric acid (main component).

MB-1 is DFNA-0078 NT, a nucleating masterbatch based on a LDPE containing 10 wt% nucleating agent. The LDPE has an MI of 2 g/10 min (ASTM D-1238, (190°C./2.16 kg)) and a density of 0.920 g/cc (ASTM D-792) available from The Dow Chemical Company.

MB-2 is NUC5532, a nucleating agent masterbatch from Clariant used as received.

MB-3 is nucleating agent masterbatch based on the low density polyethylene LDPE-2 (DFDB-1258 NT) containing 34% of HYDROCEROL™ CF.

MB-4 is hybrid nucleating agent masterbatch based on the low density polyethylene LDPE-2 (DFDB-1258 NT) containing 9.7% of PTFE and 2.3% of HYDROCEROL™ CF.

MB-5 is hybrid nucleating agent masterbatch based on the low density polyethylene LDPE-2 (DFDB-1258 NT) containing 9.0% of PTFE and 4.3% of HYDROCEROL™ CF.

MB-6 is hybrid nucleating agent masterbatch based on the low density polyethylene LDPE-2 (DFDB-1258 NT) containing 8.0% of PTFE and 8.7% of HYDROCEROL™ CF.

MB-7 is hybrid nucleating agent masterbatch based on the low density polyethylene LDPE-2 (DFDB-1258 NT) containing 6.7% of PTFE and 20% of HYDROCEROL™ CF.

MB-8 is hybrid nucleating agent masterbatch based on the low density polyethylene LDPE-2 (DFDB-1258 NT) containing 5.3% of PTFE and 28% of HYDROCEROL™ CF.

MB-9 is hybrid nucleating agent masterbatch based on the low density polyethylene LDPE-2 (DFDB-1258 NT) containing 8.0% of PTFE and 8.7% of W280.

*Endothermic Nucleating Agent*

CM-2 (i.e., W280) is a mixture of citric acid and sodium dihydrogen citrate, used as received. For CM-1 (i.e., HYDROCEROL™ CF) is also used as received. It is a specially formulated, multi-component system, and it is an endothermic-type nucleating agent.

*Preparation of Nucleating Agent Masterbatch*

The preparation of the nucleating agent masterbatch is conducted in an internal mixer The masterbatches are prepared using a BRABENDER™ model Prep Mixer/Measuring Head laboratory electric batch mixer equipped with cam blades. The Prep-Mixer® is a 3-piece design consisting of two heating zones with a capacity of 350/420mL depending on mixer-blade configuration. The formulations mixed per batch are detailed in Table 1.

Each compound is made by first adding the polyethylene resin to the mixing bowl at 120°C. The polyethylene is allowed to mix for about 5 minutes at a rpm of 35 yielding a resin in a fluxed state. The nucleating agents and antioxidants are then added to the mixer and then allowed to mix for an additional 4 minutes at 35 rpm. Once the mixing is completed, the molten material is backed out of the mixer using tweezers and collected. The molten material is then placed between two MYLAR™ sheets and compression molded at room temperature and 2500 psi pressure into a flat pancake, then cut into small pieces (approximately 0.5 cm x 0.5 cm).

Table 1

| Nucleating Agent Masterbatch Compositions | | | | | | | | | |
|---|---|---|---|---|---|---|---|---|---|
| | MB-1 | MB-2 | MB-3 | MB-4 | MB-5 | MB-6 | MB-7 | MB-8 | MB-9 |
| DFNA-0078 | 100 | | | | | | | | |
| NUC5532 | | 100 | | | | | | | |
| DFDB-1258 NT | | | 64.5 | 86.5 | 85.2 | 81.8 | 71.8 | 65.2 | 81.8 |
| MP1400, PTFE | | | | 9.7 | 9.0 | 8.0 | 6.7 | 5.3 | 8.0 |
| HYDROCEROL™ CF | | | 34 | 2.3 | 4.3 | 8.7 | 20 | 28 | |
| W280 | | | | | | | | | 8.7 |
| SONGNOX™ 1024 FG | | | 1.5 | 1.5 | 1.5 | 1.5 | 1.5 | 1.5 | 1.5 |
| Total | 100 | 100 | 100 | 100 | 100 | 100 | 100 | 100 | 100 |

*Foaming Process*

Foaming is conducted on a single-screw extruder with equipped with a gas injection system. The screw diameter is 50 millimeters (mm) with length to diameter (L/D) ratio of 40. The gas injection point is located at the middle of screw with $CO_2$ as the blowing agent. The capillary die has a diameter of 3 mm. The temperature profile is 140/175/180(gas injection)/170/145(static mixer)/143(die). HDPE-1, LDPE-2 and nucleating agent MB are dry blended first then fed on the upstream of the extruder, or DGDA-6944 and the masterbatch are compounded into "all in one" formulation and then foamed on the gas injected extruder. The exgtruded foam rod has a diameter of 13-16 mm depending on the expansion ratio of each formulation.

*Characterization of Extruded Foam Rod*

*Expansion Ratio*

The expansion ratio is calculated based on the density of sample before and after foaming. The density of the foamed article and solid plaque are measured according to ASTM D792.

$$Expansion\, ratio = \left(1 - \frac{\rho_{foam}}{\rho_{solid}}\right) * 100\%$$

*Average Cell Size*

The foamed sample is fractured utilizing liquid nitrogen and then slices are cut out using a razor blade. The slices are coated with platinum using an EMITECH™ K575X coater before scanning electron microscopy (SEM) analysis. The SEM images are acquired on a FEI Nova NanoSEM 630 SEM by Everhart-Thornley detector (ETD) and Through Lens Detector (TLD) at an accelerating voltage of 5 kV, working distance around 6.5 mm and spot size of 5. The average cell size is obtained through the analysisof the SEM photographs.
The cell density of the foamed article can be calculated by the following Equation:

$$N_f = \left(\frac{n_c M_c^2}{A_c}\right)^{3/2}$$

$N_f$ represents cell number per cubic centimeter volume in the foamed article, $n_c$ is the cell number in the view area of SEM picture, $A_c$ is the area of SEM picture, and $M_c$ is the magnification.
*D*, which is the average the cell size, can be calculated by the following Equation:

$$D = \left(\frac{6V_t^2}{\pi N_f}\right)^{1/3}$$

Where, $V_t$ represents that expansion ratio of foamed article.
DF measurements: Dissipation Factor measurements are conducted on a High Frequency Split Post Dielectric Resonator at a frequency of 2.47GHz on 50 mil compression molded plaques. Before measurements, the plaques are conditioned for 24 hours at room temperature in a desiccant chamber.
The results are reported in Table 2.

Table 2

| | CE-1 | CE-2 | CE-3 | CE-4 | IE-1 | IE-2 | IE-3 | IE-4 | IE-5 | IE-6 |
|---|---|---|---|---|---|---|---|---|---|---|
| Foaming Performance of Hybrid Nucleating Agents | | | | | | | | | | |
| Component | | | | | | | | | | |
| HDPE-1 | 70 | 70 | 70 | 70 | 70 | 70 | 70 | 70 | 70 | 70 |
| LDPE-2 | 28.5 | 28.5 | 25 | 28.5 | 28.5 | 28.5 | 28.5 | 28.5 | 28.5 | 28.5 |
| MB-2 | 1.5 | | | | | | | | | |
| MB-1 | | 1.5 | 5.0 | | | | | | | |
| MB-3 | | | | 1.5 | | | | | | |
| MB-4 | | | | | 1.5 | | | | | |
| MB-5 | | | | | | 1.5 | | | | |
| MB-6 | | | | | | | 1.5 | | | |
| MB-7 | | | | | | | | 1.5 | | |
| MB-8 | | | | | | | | | 1.5 | |
| MB-9 | | | | | | | | | | 1.5 |
| Total | 100 | 100 | 100 | 100 | 100 | 100 | 100 | 100 | 100 | 100 |
| Nucleating agent powder loading level in the final expandable compound, PPM | | | | | | | | | | |
| PTFE, PPM | | 1500 | 5000 | | 1450 | 1350 | 1200 | 1000 | 800 | 1200 |
| CM-1, PPM | | | | 5000 | 350 | 650 | 1300 | 3000 | 4200 | |
| CM-2, PPM | | | | | | | | | | 1300 |
| Total | 1500 | 1500 | 5000 | 5000 | 1800 | 2000 | 2500 | 4000 | 5000 | 2500 |
| Foaming Performance | | | | | | | | | | |
| Expansion ratio, % | 84.3 | 77.1 | | 80.7 | 77.5 | 78.9 | 79.4 | 79.6 | 80.3 | 77.5 |
| Average cell size, um | 406 | 478 | 393 | 357 | 469 | 394 | 385 | 344 | 329 | 355 |

(continued)

| | | | | | | | | | | |
|---|---|---|---|---|---|---|---|---|---|---|
| **Foaming Performance** | | | | | | | | | | |
| Water residue in foamed part, ppm | 273 | 58 | 22 | 305 | 87 | 126 | 167 | 218 | 277 | 114 |
| DF of nucleating agent MB plaque | 7.9393E-04 | 4.8706E-04 | 4.8706E-04 | 1.2403E-03 | Progress | 5.0640E-04 | 6.5101 E-04 | 8.8371 E-04 | 1.0863E-03 | 7.0587E-04 |
| Surface smoothness rating | ++ | - | + | ++ | - | + | ++ | +++ | +++ | +++ |

*Results*

The results show that the combination of PTFE with a mixture of (i) citric acid and/or sodium citrate, and (ii) one or more of its derivatives (e.g., sodium citrate, disodium hydrogen citrate, sodium dihydrogen citrate) and/or sodium bicarbonate (CM-1 or CM-2) has better foaming performance, e.g. finer cell size and smoother surface, than neat PTFE or a neat mixture of sodium citrate and bicarbonate. At an equivalent or lower loading of nucleating agent, the inventive process shows improved foaming performance, e.g., higher expansion ratio and smaller cell size, or lower DF of nucleating agent plaques.

However, when the loading level of CM-1 is lower than 0.03% in the final compound (Example 1), no desired benefit on foaming performance is seen as compared to the use of PTFE alone. So the lower limit of the mixture of sodium citrate and bicarbonate is 300 parts per million (ppm) in the final compound. The foaming became worse when the loading of CM-1is higher than 5000 ppm (Comparative Example 4) in the final compound. This can be attributed to the severe cell coalescence due to the excessive released gas. The excessive loading of CM-1 also leads to a higher DF value which increases the signal transmission attenuation. With increasing the ratio of CM-1 or CM-2 to PTFE, the foaming is improved.

**Claims**

**1.** A process of foaming a polyolefin composition using as a nucleator a combination of (A) a fluororesin, and (B) a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate, wherein the polyolefin of the polyolefin composition comprises high density polyethylene (HDPE) and low density polyethylene (LDPE) and wherein the fluororesin is present in the composition in an amount of 0.01 to 1 wt% based on the weight of the composition, and the mixture is present in the composition in an amount of 300 to 5000 parts per million (ppm).

**2.** The process of Claim 1 in which the polyolefin of the polyolefin composition consists of HDPE and LDPE.

**3.** The process of Claim 1 in which the HDPE comprises 45 to 95 weight percent of the composition based on the weight of the composition and the LDPE comprises 4 to 54 weight percent of the composition based on the weight of the composition.

**4.** The process of Claim 1 in which the fluororesin comprises polytetrafluoroethylene (PTFE).

**5.** The process of Claim 2 in which the fluororesin is PTFE.

**6.** The process of Claim 5 in which the composition further comprises at least one of an antioxidant and a cell stabilizer.

**7.** A foam made by a process of the any of Claims 1-6.

**8.** A cable comprising an insulation layer comprising the foam of Claim 7.

**9.** A foamable composition comprising in weight percent based on the weight of the composition:

(A) 45 to 95% HDPE;
(B) 4 to 54% LDPE;
(C) 0.01 to 1% of PTFE; and
(D) 300 to 5000 ppm of a mixture of (1) a first component consisting of at least one of citric acid and an alkali metal citrate, and (2) a second component consisting of at least one of an alkali metal citrate, a di-alkali metal hydrogen citrate, an alkali metal dihydrogen citrate and an alkali metal bicarbonate with the proviso that if the first component of the mixture is an alkali metal citrate, then the second component of the mixture is not an alkali metal citrate.

**10.** The foamable composition of claim 9, wherein a blend of the HDPE and the LDPE has a melt index, $I_2$, from 0.1 g/10 min to 4 g/10 min.

**Patentansprüche**

1. Ein Verfahren zum Schäumen einer Polyolefinzusammensetzung unter Verwendung, als Keimbildner, einer Kombination aus (A) einem Fluorharz und (B) einer Mischung aus (1) einem ersten Bestandteil, der aus mindestens einem von Zitronensäure und einem Alkalimetallcitrat besteht, und (2) einem zweiten Bestandteil, der aus mindestens einem von einem Alkalimetallcitrat, einem di-Alkalimetallhydrogencitrat, einem Alkalimetalldihydrogencitrat und einem Alkalimetallbicarbonat besteht, mit der Maßgabe, dass dann, wenn der erste Bestandteil der Mischung ein Alkalimetallcitrat ist, der zweite Bestandteil der Mischung kein Alkalimetallcitrat ist, wobei das Polyolefin der Polyolefinzusammensetzung Polyethylen hoher Dichte (High Density Polyethylene, HDPE) und Polyethylen niederer Dichte (Low Density Polyethylene, LDPE) beinhaltet und wobei das Fluorharz in der Zusammensetzung in einer Menge von 0,01 bis 1 Gew.-%, bezogen auf das Gewicht der Zusammensetzung, präsent ist und die Mischung in der Zusammensetzung in einer Menge von 300 bis 5000 Teilen pro Million (ppm) präsent ist.

2. Verfahren gemäß Anspruch 1, wobei das Polyolefin der Polyolefinzusammensetzung aus HDPE und LDPE besteht.

3. Verfahren gemäß Anspruch 1, wobei das HDPE 45 bis 95 Gewichtsprozent der Zusammensetzung, bezogen auf das Gewicht der Zusammensetzung, beinhaltet und das LDPE 4 bis 54 Gewichtsprozent der Zusammensetzung, bezogen auf das Gewicht der Zusammensetzung, beinhaltet.

4. Verfahren gemäß Anspruch 1, wobei das Fluorharz Polytetrafluorethylen (PTFE) beinhaltet.

5. Verfahren gemäß Anspruch 2, wobei das Fluorharz PTFE ist.

6. Verfahren gemäß Anspruch 5, wobei die Zusammensetzung ferner mindestens eines von einem Antioxidationsmittel und einem Zellstabilisator beinhaltet.

7. Ein Schaum, der durch ein Verfahren gemäß einem der Ansprüche 1-6 hergestellt wird.

8. Ein Kabel, beinhaltend eine Isolierschicht, die den Schaum gemäß Anspruch 7 beinhaltet.

9. Eine schäumbare Zusammensetzung, die in Gewichtsprozent, bezogen auf das Gewicht der Zusammensetzung, Folgendes beinhaltet:

   (A) zu 45 bis 95 % HDPE;
   (B) zu 4 bis 54 % LDPE;
   (C) zu 0,01 bis 1 % PTFE; und
   (D) zu 300 bis 5000 ppm eine Mischung aus (1) einem ersten Bestandteil, der aus mindestens einem von Zitronensäure und einem Alkalimetallcitrat besteht, und (2) einem zweiten Bestandteil, der aus mindestens einem von einem Alkalimetallcitrat, einem di-Alkalimetallhydrogencitrat, einem Alkalimetalldihydrogencitrat und einem Alkalimetallbicarbonat besteht, mit der Maßgabe, dass dann, wenn der erste Bestandteil der Mischung ein Alkalimetallcitrat ist, der zweite Bestandteil der Mischung kein Alkalimetallcitrat ist.

10. Schäumbare Zusammensetzung gemäß Anspruch 9, wobei ein Gemisch des HDPE und des LDPE einen Schmelzindex, $I_2$, von 0,1 g/10 min bis 4 g/10 min aufweist.

**Revendications**

1. Un procédé de moussage d'une composition de polyoléfine utilisant en tant qu'agent de nucléation une combinaison (A) d'une résine fluorée, et (B) d'un mélange (1) d'un premier constituant consistant en au moins un élément parmi de l'acide citrique et un citrate de métal alcalin, et (2) d'un deuxième constituant consistant en au moins un élément parmi un citrate de métal alcalin, un hydrogénocitrate de métal di-alcalin, un dihydrogénocitrate de métal alcalin et un bicarbonate de métal alcalin à la condition que si le premier constituant du mélange est un citrate de métal alcalin, alors le deuxième constituant du mélange ne soit pas un citrate de métal alcalin, où la polyoléfine de la composition de polyoléfine comprend du polyéthylène haute densité (HDPE) et du polyéthylène basse densité (LDPE) et où la résine fluorée est présente dans la composition dans une quantité de 0,01 à 1 % en poids rapporté au poids de la composition, et le mélange est présent dans la composition dans une quantité de 300 à 5 000 parties par million (ppm).

**2.** Le procédé de la revendication 1 dans lequel la polyoléfine de la composition de polyoléfine consiste en HDPE et LDPE.

**3.** Le procédé de la revendication 1 dans lequel le HDPE constitue 45 à 95 pour cent en poids de la composition rapporté au poids de la composition et le LDPE constitue 4 à 54 pour cent en poids de la composition rapporté au poids de la composition.

**4.** Le procédé de la revendication 1 dans lequel la résine fluorée comprend du polytétrafluoroéthylène (PTFE).

**5.** Le procédé de la revendication 2 dans lequel la résine fluorée est le PTFE.

**6.** Le procédé de la revendication 5 dans lequel la composition comprend en outre au moins un élément parmi un antioxydant et un stabilisateur de cellules.

**7.** Une mousse fabriquée par un procédé de n'importe lesquelles des revendications 1 à 6.

**8.** Un câble comprenant une couche isolante comprenant la mousse de la revendication 7.

**9.** Une composition moussable comprenant en pourcentage en poids rapporté au poids de la composition :

(A) de 45 à 95 % de HDPE ;
(B) de 4 à 54 % de LDPE ;
(C) de 0,01 à 1 % de PTFE ; et
(D) de 300 à 5 000 ppm d'un mélange (1) d'un premier constituant consistant en au moins un élément parmi de l'acide citrique et un citrate de métal alcalin, et (2) d'un deuxième constituant consistant en au moins un élément parmi un citrate de métal alcalin, un hydrogénocitrate de métal di-alcalin, un dihydrogénocitrate de métal alcalin et un bicarbonate de métal alcalin à la condition que si le premier constituant du mélange est un citrate de métal alcalin, alors le deuxième constituant du mélange ne soit pas un citrate de métal alcalin.

**10.** La composition moussable de la revendication 9, où un mélange homogène du HDPE et du LDPE a un indice de fusion, $I_2$, allant de 0,1 g/10 min à 4 g/10 min.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3554932 A
- US 20090018225 A
- CA 2523861 A1
- US 4302565 A
- US 4508842 A
- US 5332793 A
- US 5342907 A
- US 5410003 A
- US 4101445 A
- US 4937299 A
- US 5317036 A
- US 5371145 A
- US 5405901 A
- US 6121335 A
- CA 2523861 C
- US 6284810 B
- US 684810 B1

**Non-patent literature cited in the description**

- **STILLE.** Introduction to Polymer Chemistry. Wiley and Sons, 1962, 149-151
- Polyolefin Foam. **C.P. PARK.** Handbook of Polymer Foams and Technology. Hanser Publishers, 1991